# EUROPEAN PATENT APPLICATION

(11) **EP 1 870 127 A1**
(43) Date of publication of application: **26.12.2007**
(21) Application number: 06425416.2
(22) Date of filing: 19.06.2006
(51) Int. Cl.: A61N 1/05

(54) **Electrical catheter for use in neurostimulation with adaptable neurostimulating tip**

(71) Applicant: Lifestim S.r.l., 35030 Rubano (PD) (IT)
(72) Inventor: Snichelotto, Eugenio c/o Lifestim S.r.l., 35030 Rubano (PD) (IT)
(74) Representative: Vinci, Marcello

(57) **Abstract**

The invention is a new electrical catheter for neurostimulation with adaptable neurostimulating tip provided with filiform body, comprising on the neurostimulating tip one or more grooves or channels suitable for permitting bending of the tip. In particular said grooves or channels are positioned between the neurostimulation terminals so that some of said terminals are positioned in areas of the tip separated from one another by said grooves or channels and not lying on the same plane. The neurostimulating tip can be provided with three or more neurostimulation electrodes connected, via insulated electrical leads, to corresponding electrical connection terminals present at the opposite end, called connection end.

## Description

The present patent concerns implantable analgesic neurostimulation devices and in particular refers to the electrodes of said implantable neurostimulation devices.

Neurostimulation devices are known, for example neurostimulators.

Neurostimulation devices are also profitably used for the treatment of chronic pain.

It is known that analgesic electrostimulation neutralises or in any case alleviates the sensation of pain, especially in cases of chronic pain.

The neurostimulation systems for the treatment of pain are electronic devices which stimulate one or more nerve fibres.

Said stimulation induces in the user a sensation of "touching" instead of a sensation of pain.

In cases of chronic pain, implantable neurostimulation devices, similar to pacemakers, which stimulate one or more nerve fibres, are applied.

A neurostimulation device substantially comprises a battery or accumulator of electrical energy, a control and command circuit that modulates the nervous stimulation, and one or more electrical catheters which perform stimulation of the nerve bundles.

The known electrical catheters are provided at one end with two sets of stimulation electrodes suitable for stimulating the receiver nerve bundle, and at the other end with two sets of other contact terminals for connection with the control and command circuit. The end provided with the stimulation terminals is generically flat, substantially rigid and presents said contact terminals on one side of said generically flat form.

The point of the nerve bundle at which it is appropriate to administer the neurostimulation is not always flat enough to obtain optimal contact between the nerve bundle itself and the stimulation terminals of the electrical catheter.

To remedy this drawback the stimulation terminals of the electrical catheter are applied to another point of the nerve bundle and/or in an optimal position for contact, but the required electrostimulation effect is not obtained. It should also be considered that the neurostimulation tip of the known electrical catheters, however small it may be, is difficult to adapt to curved application surfaces.

Application of the terminal of the electrical catheter requires high precision, both in order not to discharge electrical current at points where it is not required, and to adequately apply the electrical impulse on the receiver nerve bundle and therefore obtain maximum stimulation efficiency.

Some types of treatment entail simultaneous or consecutive stimulation of the same or several nerve bundles.

Each electrical catheter implanted is a foreign body inside the human body, which, even if biocompatible, can always cause disorders and complications.

The minimum application distance between the terminals of the electrical catheters applied adjacent depends on the overall dimension of the electrical catheters and in various cases is greater than necessary for correct treatment.

Continuous electrostimulation of a point of the nerve bundle can lead to habituation of the nerve bundle to the electrostimulation with the result that the effectiveness of the electrostimulation impulses is reduced.

To remedy all the above drawbacks a new electrical catheter for neurostimulation with adaptable neurostimulating tip has been designed and produced.

The object of the new electrical catheter for neurostimulation is to permit the adaptation of its neurostimulating tip to particularly curved stimulation surfaces and/or surfaces with particularly narrow radius of curvature.

A further object of the new neurostimulation electrical catheter is to permit adaptation of the neurostimulating tip around the nerve bundle to be stimulated.

A further object of the new neurostimulation electrical catheter is to improve the contact between the neurostimulation electrodes of the catheter itself and the surface of the nerve bundle to be stimulated.

A further object of the new electrical catheter for neurostimulation is to permit positioning of the electrodes nearer and in contact with the nerve bundle so that less energy is required for the neurostimulation and the scheduled life of the neurostimulation device is prolonged.

A further object of the new electrical catheter for neurostimulation is to permit stimulation in points or areas of the nerve bundles that are very near to one another.

A further object of the new electrical catheter for neurostimulation is to obtain reduced overall dimensions.

A further object of the new electrical catheter for neurostimulation is to reduce the number of connections that have to be made between the neurostimulation device and the electrical catheters.

A further object of the new electrical catheter for neurostimulation is to reduce habituation to electrostimulation.

These and other direct and complementary objects have been achieved through the implementation of the new electrical catheter for neurostimulation with adaptable neurostimulating tip provided with a filiform body comprising on the neurostimulating tip one or more grooves or channels suitable for permitting bending of the tip.

In particular said grooves or channels are positioned between the neurostimulation terminals so that some of said terminals are in areas of the tip that are separated from one another by said grooves or channels and not lying on the same plane.

It is possible to provide the neurostimulating tip with three or more neurostimulation electrodes connected, via insulated electrical leads, to corresponding electrical connection terminals present at the opposite end, called connection end.

In this case the neurostimulation electrodes of the neurostimulating tip can be arranged in any pattern, preferably according to an arrangement on two rows of alternate electrodes on two sides of the neurostimulating tip and separated by at least one groove or channel.

The connection terminals of the connection end can be arranged in any way, preferably according to an arrangement in succession on one side or on the circumference of the filiform body.

The filiform body can be made of any insulating flexible biocompatible material.

The connection terminals and the neurostimulation electrodes can be made of any suitable conductive biocompatible material, for example titanium, platinum/iridium, platinum-coated titanium.

The characteristics of the new electrical catheter for neurostimulation with adaptable neurostimulating tip will be highlighted in greater detail in the following description with reference to the drawings attached as non-limiting examples.

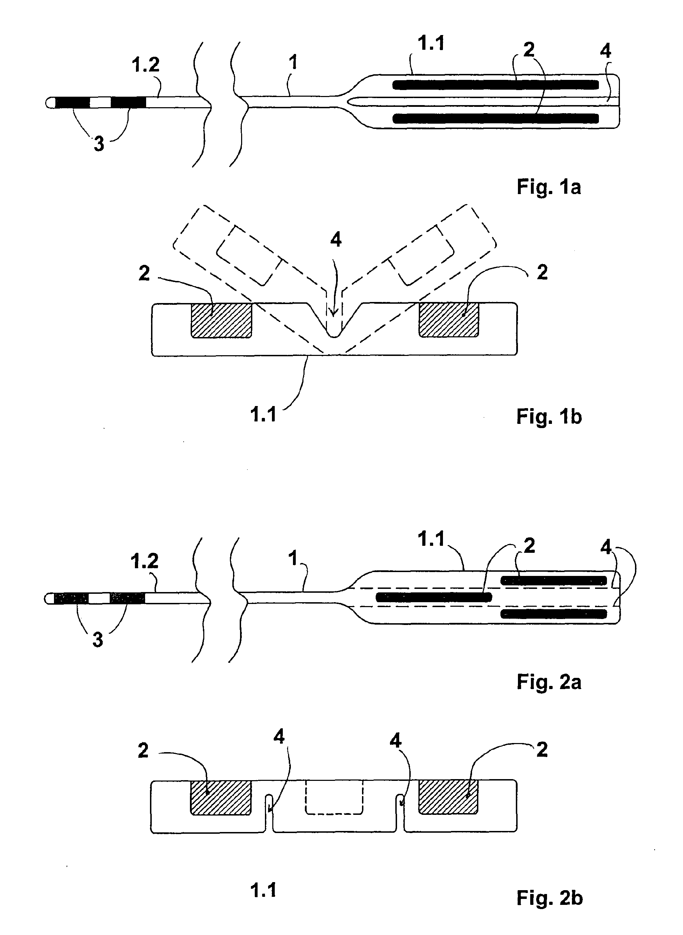

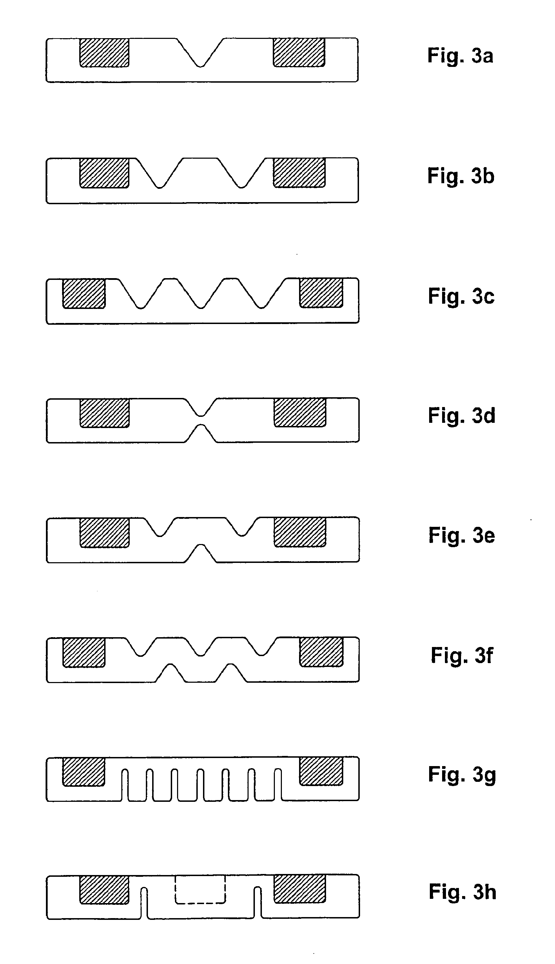

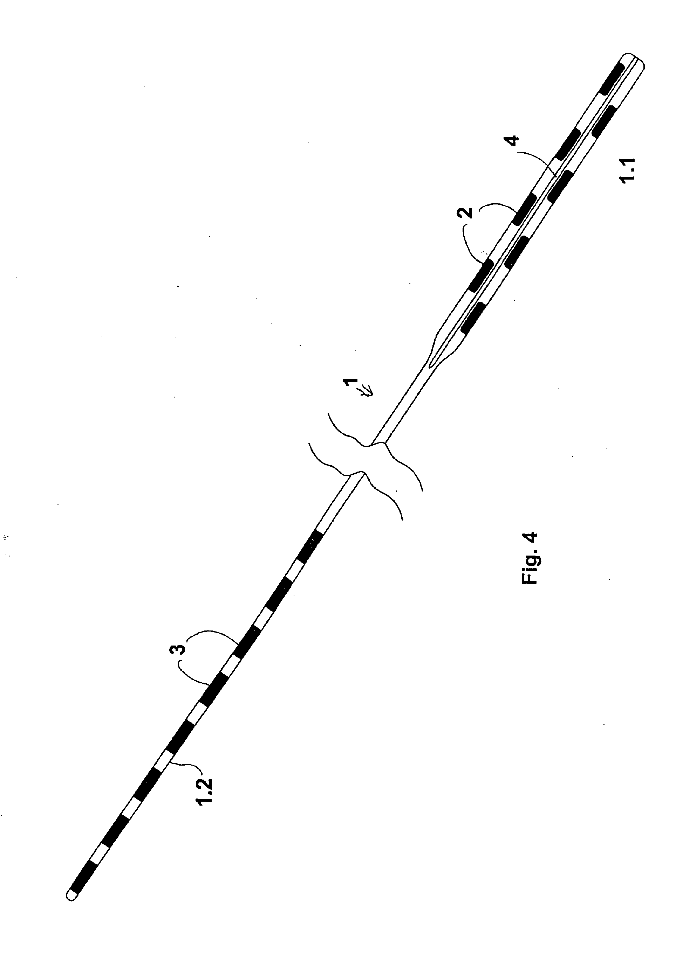

Figure 1 is a schematic view of the new electrical catheter, while Figure 1b shows a section of the neurostimulating tip of the new electrical catheter.

The new electrical catheter for neurostimulation comprises a filiform body (1) having at one end (1.1), called neurostimulating tip, two or more neurostimulation electrodes (2) and at the opposite end (1.2), called connection end, terminals or electrical contacts (3) for connection to the neurostimulation device, not shown in the figure.

Said connection terminals (3) are connected, via appropriate electrical connections inside the body (1) of the new electrical catheter, to the neurostimulation electrodes (2). The body (1) of the new electrical catheter for neurostimulation has a linear shape with reduced thickness and is made of flexible biocompatible material, preferably silicone or polyurethane specific for body implants. Said body (1) of the electrical catheter can have a constant section throughout its length or sections of different width at each end (1.1, 1.2) and/or in its central portion.

At the neurostimulating tip (1.1) of the body (1) of the new electrical catheter there are two or more neurostimulation electrodes (2).

Said neurostimulating tip has a groove or channel (4) positioned between said neurostimulation electrodes (2).

Said groove, channel or other (4) is such as to reduce the thickness of the neurostimulating tip (1.1) along a flexion line and divide it into two parts (1.1 a, 1.1 b) that can rotate around each other.

The two neurostimulation electrodes (2) are thus divided between the two parts of the neurostimulating tip (1.1) that can rotate around each other (1.1 a, 1.1b) so as to lie on non-parallel planes.

In this way said neurostimulating tip (1.1) can be easily adapted and wrapped around the nerve bundle to be stimulated, arranging the neurostimulation electrodes (2) appropriately in contact with said nerve bundle.

The Figures from 3a to 3h illustrate in section, as a non-limiting example, various examples of embodiments of the neurostimulating tip (1.1). In particular in said figures it is possible to observe some examples of the quantity and arrangement of the grooves or channels (4) suitable for making the neurostimulating tip more flexible and more adaptable to application to very curved surfaces.

Figures 2a and 2b show a second embodiment of the new electrical catheter.

In this example there are three neurostimulation electrodes (2) arranged offset and two grooves or channels (4) generically aligned with the length of the new electrical catheter such as to separate the neurostimulating tip (1.1) into three parts (1.1a, 1.1b, 1.1c) each of which contains an electrode (2). In this case two of the three neurostimulation electrodes (2) are connected together electrically so as to improve neurostimulation.

It is possible to provide the neurostimulating tip with several neurostimulation terminals (2).

Figure 4 shows a further embodiment of the invention, in which several neurostimulation electrodes (2) are present.

In this example there are eight different neurostimulation electrodes (2), arranged in two offset series on two opposite sides of the neurostimulating tip (1.1); a channel or groove (4) separates said two series of electrodes (2).

Electrical connection terminals (3) are present at the connection end (1.2).

Said connection terminals (3) are connected, via appropriate electrical connections inside the body (1) of the new electrical catheter, to the neurostimulation electrodes (2).

It is preferable for said connection terminals (3) to be present in the same number as the number of neurostimulation electrodes (2), so that each connection terminal (3) is connected to a corresponding neurostimulation electrode (2).

The connection terminals (3) can be arranged in any way on the connection end (1.2) of the new electrical catheter, and are preferably arranged in succession on one side or on the circumference of the filiform body (1).

Both the neurostimulation electrodes (2) and the connection terminals (3) are made of any suitable conductive biocompatible material, for example titanium, platinum/iridium, platinum-coated titanium.

The new electrical catheter for neurostimulation with adaptable neurostimulating tip and multiple electrodes constituted as described above offers considerable advantages.

The channels or grooves permit flexion of the neurostimulating tip (1.1), adapting it to the particularly curved surfaces of the nerve bundle to be stimulated.

The flexion or bending of the neurostimulating tip (1.1) improves the contact between the neurostimulation electrodes (2) and the surface of the nerve bundle to be stimulated.

Said improved contact between neurostimulation electrodes (2) and nerve bundle allows for reduction of the intensity of the stimulation impulses, reduction of the energy required for the neurostimulation and consequently increase in the available operating time of the neurostimulation device.

The new electrical catheter for neurostimulation permits stimulation in points or areas of the nerve bundles that are very close to one another.

The new electrical catheter for neurostimulation has reduced overall dimensions with the same number of neurostimulation electrodes, consequently the invasiveness due to the presence of the new electrical catheter is very limited.

The new electrical catheter for neurostimulation requires one or in any case few connections between the neurostimulation device and the electrical catheter. This reduces the application or assembly time and the possibility of making inaccurate connections.

The new electrical catheter for neurostimulation, by applying the electrical stimulation alternatively to different points of the same nerve bundle, considerably reduces habituation of the nerve bundle to the electrostimulation.

## Claims

1. Electrical catheter for neurostimulation, comprising a filiform body (1) having a neurostimulating tip (1.1) with at least two neurostimulation electrodes (2) and a connection end (1.2), **characterised in that** it has one or more channels or grooves (4) on the neurostimulating tip (1.1) to ensure flexibility of said neurostimulating tip (1.1), and wherein at least one of said grooves or channels (4) is arranged between the neurostimulation electrodes (2).

2. Electrical catheter for neurostimulation according to the preceding claim, **characterised in that** said grooves or channels (4) are generically parallel to the length of the electrical catheter.

3. Electrical catheter for neurostimulation according to the preceding claims, **characterised in that** said grooves or channels (4) are present on one or both the opposite sides of the neurostimulating tip (1.1).

4. Electrical catheter for neurostimulation according to the preceding claims, **characterised in that** said channels or grooves (4) have a generically V-shaped section.

5. Electrical catheter for neurostimulation according to claims 1, 2, 3, **characterised in that** said channels or grooves (4) have a generically linear or I-shaped section.

6. Electrical catheter for neurostimulation according to claims 1, 2, 3, **characterised in that** said channels or grooves (4) have a generically semicircular or semi-elliptic section.

7. Electrical catheter for neurostimulation according to the preceding claims, **characterised in that** it has two or more connection terminals (3) on the connection end (1.2), and wherein each of said connection terminals (3) is electrically connected to one or more neurostimulation electrodes (2).

8. Electrical catheter for neurostimulation according to the preceding claims, **characterised in that** said neurostimulation electrodes (2) are arranged on one single side of the neurostimulating tip (1.1).

9. Electrical catheter for neurostimulation according to claims 1, 2, 3, 4, 5, 6, 7, **characterised in that** said neurostimulation electrodes (2) are arranged on two or more sides of the neurostimulating tip (1.1).

10. Electrical catheter for neurostimulation according to the preceding claim, **characterised in that** said neurostimulation electrodes (2) are arranged alternately on the sides of the neurostimulating tip (1.1).

11. Electrical catheter for neurostimulation according to claims 1, 2, 3, 4, 5, 6, 7, **characterised in that** said neurostimulation electrodes (2) have an annular shape and are arranged in succession on the neurostimulating tip (1.1).

12. Electrical catheter for neurostimulation according to claims 1, 2, 3, 4, 5, 6, 7, **characterised in that** said connection terminals (3) are arranged in succession on the circumference of the connection end.

13. Electrical catheter for neurostimulation according to claims 1, 2, 3, 4, 5, 6, 7, **characterised in that** said connection terminals (3) are arranged in succession on one side of the connection end (1.2).

14. Electrical catheter for neurostimulation according to the preceding claims, **characterised in that** said filiform body (1) of the electrical catheter is made of flexible biocompatible material.

15. Electrical catheter for neurostimulation according to the preceding claim, **characterised in that** said filiform body (1) of the electrical catheter is made of biocompatible silicone.

16. Electrical catheter for neurostimulation according to claim 14, **characterised in that** said filiform body (1) of the electrical catheter is made of biocompatible polyurethane.

17. Electrical catheter for neurostimulation according to claims 1, 2, 3, 4, 5, 6, 7, 8, 9, 10, 11, 12, 13, **characterised in that** said connection terminals (3) and/or said neurostimulation electrodes (2) are made of titanium.

18. Electrical catheter for neurostimulation according to claims 1, 2, 3, 4, 5, 6, 7, 8, 9, 10, 11, 12, 13, **characterised in that** said connection terminals (3) and/or said neurostimulation electrodes (2) are made of platinum and iridium.

19. Electrical catheter for neurostimulation according to claims 1, 2, 3, 4, 5, 6, 7, 8, 9, 10, 11, 12, 13, **characterised in that** said connection terminals (3) and/or said neurostimulation electrodes (2) are made of platinum-coated titanium.
